# FASCICULE DE BREVET EUROPEEN

(11) **EP 0 787 180 B1**
(45) Date de publication et mention de la délivrance du brevet: **04.09.2002**
(21) Numéro de dépôt: 95935994.4
(22) Date de dépôt: 20.10.1995
(51) Int. Cl.: C12N 5/06, C12N 5/10, A01K 67/027

(54) **MILIEU DE CULTURE DE CELLULES EMBRYONNAIRES TOTIPOTENTES AVIAIRES, DEPOURVU D'ACIDE RETINOIQUE ACTIF**
KULTURMEDIUM FÜR TOTIPOTENTE EMBRYONALEN VOGELZELLEN DAS VON AKTIVER RETINOINSÄURE FREI IST
ACTIVE RETINOIC ACID FREE CULTURE MEDIUM FOR AVIAN TOTIPOTENTIAL EMBRYONIC CELLS

(30) Priorité: 21.10.1994 FR 9412598
(43) Date de publication de la demande: 06.08.1997
(60) Demande divisionnaire: 01108584.2
(73) Titulaire: INSTITUT NATIONAL DE LA RECHERCHE AGRONOMIQUE (INRA), 75007 Paris Cédex 07 (FR); CENTRE NATIONAL DE LA RECHERCHE SCIENTIFIQUE, 75016 Paris Cédex (FR); ECOLE NORMALE SUPERIEURE DE LYON, 69007 Lyon Cédex 07 (FR)
(72) Inventeur: SAMARUT, Jacques, F-69100 Villeurbanne (FR); PAIN, Bertrand, F-69003 Lyon (FR)
(74) Mandataire: Ahner, Francis
(86) Numéro de dépôt international: FR9501389
(87) Numéro de publication internationale: WO96012793

(56) Documents cités:
- WO-A-90/01541
- WO-A-93/15185
- WO-A-93/23528
- DATABASE MEDLINE FILE SERVER STN KARLSRUHE ABR G 87191399, SMITH ET AL 'BUFFALO RAT LIVER CELLS PRODUCE A DIFFUSIBLE ACTIVITY WHICH INHIBITS THE DIFFERENTIATION OF MURINE EMBRYONAL CARCINOMA AND EMBRYONIC STEM CELLS'
- DATABASE MEDLINE FILE SERVER STN KARLSRUHE ABR G 93365092, SLAGER ET AL 'TRANSFORMING GROWTH FACTOR-BETA IN THE EARLY MOUSE EMBRYO:IMPLICATIONS FOR THE REGULATION OF MUSCLE FORMATION AND IMPLANTATION'
- R.IAN FRESHNEY 'SECOND EDITION.CULTURE OF ANIMAL CELLS.A MANUAL OF BASIC TECHNIQUE' 1987 , ALAN R.LISS,INC. , NEW YORK voir page 187 - page 196

## Description

La présente invention se rapporte à l'obtention de cellules ES d'oiseau, en particulier à un procédé de culture et à un milieu permettant la culture de ces cellules.

En effet, dans le cadre de la mise au point de technique de production de protéines recombinantes, le développement d'une technique de transgénèse chez les oiseaux domestiques aura des retombées économiques extrêmement importantes dans deux applications majeures:
- 1. le développement de souches aviaires présentant des caractères génétiques déterminés (résistance à certaines maladies, performances de croissance, etc)
- 2. le développement de systèmes de production de protéines recombinantes dans l'albumen de l'oeuf.

L'industrie biotechnologique s'intéresse de plus en plus à la possibilité de produire des protéines d'intérêt dans des fluides biologiques ou des organismes (sang, lait, plantes, ...). La production de telles protéines dans l'oeuf d'oiseau domestique constituera certainement dans cette voie une avancée technologique majeure pour plusieurs raisons:
- de nombreuses protéines de mammifères ne peuvent être produites en système mammifère car leur surabondance dans ces organismes présente des effets délétères (exemple: l'érythropoiétine qui chez le lapin induit des hyperglobulinémies pathologiques). Beaucoup de ces protéines d'intérêt ne présentent pas d'activité croisée avec celles des oiseaux, autorisant ainsi leur surproduction dans un organisme aviaire sans effet pathologique majeur;
- il est très vraisemblable que la commercialisation de protéines recombinantes produites chez des mammifères se heurtera à des problèmes sanitaires liés à la présence chez cette espèces d'organismes latents potentiellement pathogènes pour l'Homme (lentivirus, prions,...). Ce risque est très minime, pour ne pas dire quasiment inexistant, pour des agents pathogènes des oiseaux domestiques;
- l'oeuf constitue un "tissu" très dense en un petit nombre de protéines. Par exemple la protéine majeure de l'oeuf d'oiseau, l'ovalbumine représente 54 % des protéines du blanc d'oeuf, soit un poids sec moyen par oeuf de 2 grammes de matière sèche environ. On peut raisonablement imaginer produire par oeuf au moins 10% de cette masse en protéine recombinante. La rentabilité économique apparait très grande si l'on considère qu'une poule pond en moyenne 2 oeufs tous les trois jours, et cette rentabilité apparait très supérieure à celle de grands mammifères si l'on considère les coûts d'élevage bien moindres des oiseaux domestiques.

La réalisation d'oiseaux transgéniques est possible actuellement avec un coût extrêmement élevé à cause de sa très faible efficacité. En effet chez les oiseaux la technique de microinjection d'ADN dans l'oeuf est quasiment impossible. D'autre part l'utilisation du système des rétrovirus vecteurs, le seul sytème efficace à ce jour, reste complexe et se heurtera certainement à une réticence de la part des industriels pour des raisons sanitaires.

Une avancée très importante à la réalisation d'animaux transgéniques a été apportée chez la souris par le développement de la technologie des cellules ES.

Les cellules ES (pour Embryonic Stem cells) sont des cellules embryonnaires totipotentes capables de régénérer tous les tissus de l'embryon, y compris le tissu germinal, après leur injection dans des embryons très précoces. Ces cellules peuvent donc être considérées comme des chevaux de Troie pour introduire de nouvelles informations génétiques dans le patrimoine génétique d'un animal. La possibilité de cultiver ces cellules à long terme in vitro, offre la possibilité d'exercer de nombreux contrôles avant leur implantation in vivo. D'autre part ces cellules peuvent être conservées de façon illimitée dans l'azote liquide, ce qui constitue une possibilité de stockage d'un patrimoine génétique.

L'utilisation de cellules ES constitue aujourdhui chez les oiseaux domestiques la voie la plus prometteuse pour la réalisation efficace d'animaux transgéniques. Des travaux récents d'un groupe canadien (R. Etches à la station de Guelph) ont suggéré que des cellules ES doivent exister dans l'embryon d'oiseau (Petitte et al., 1990). Ce groupe à réussi la transplantation de telles cellules dans des embryons, et par suite, la production d'animaux dont le patrimoine génétique est dérivé des cellules greffées. Cependant à ce jour la culture de ces cellules in vitro n'a pas pu être réussie ; par conséquent ces cellules n'ont pas pu être utilisées pour transférer de façon stable un transgène. C'est là un blocage majeur à l'exploitation de la technologie des cellules ES chez les oiseaux. Les cellules ES peuvent être caractérisées par trois types de critères essentiels :
- morphologie
- activité phosphatase alcaline endogène
- réaction avec des anticorps spécifiques d'un état de totipotence (ECMA-7, SSEA-1 et SSEA-3 notamment).

Aucune culture de cellules ES identifiées par l'ensemble de ces caractéristiques n'a pu être obtenue à ce jour.

C'est pourquoi la présente invention a pour objet un milieu de culture de cellules embryonnaires totipotentes aviaires du type comportant un milieu de culture pour cellules aviaires, caractérisé en ce qu'il comprend au moins un composé choisi parmi les cytokines, les facteurs de croissance des fibroblastes, les facteurs de croissance analogue de l'insuline, les facteurs de croissance des cellules souches,
et en ce que l'acide rétinoïque est inactivé par des anticorps anti-acide rétinoïque (ARMA) présents dans le milieu.

En effet, les milieux employés contiennent souvent du sérum, dont on ne peut contrôler la quantité endogène d'acide rétinoïque. En testant l'effet de l'incorporation au milieu de culture d'un anticorps monoclonal anti-acide rétinoïque qui neutraliserait l'action de ce dernier, sur la différenciation des cellules, la Demanderesse a constaté que la présence de cet anticorps accroit la présence dans les cultures de cellules et colonies à activité phosphatase alcaline.

La cytokine peut notamment être choisie parmi le LIF, IL-11, IL-6, CNTF et oncostatine M (OSM) ; de manière avantageuse les cytokines présentes dans le milieu de culture décrit précédemment, comprennent au moins une cytokine choisie dans le groupe constitué de LIF, IL-11, IL-6, et leurs différents mélanges, qui donnent les meilleurs résultats de stimulation de croissance.

De préférence, le facteur de croissance des fibroblastes est le b- FGF (ou basic Fibroblast Growth Factor) et le facteur de croissance analogue de l'insuline est l'IFG-1.

Le facteur de croissance des cellules souches · (ou SCF) est de préférence l'a-SCF (ou avian Stem Cell Factor) et le m-SCF (ou murine Stem Cell Factor).

L'un des aspects préférés de l'invention concerne un milieu de culture qui contient, outre les éléments nutritifs de base nécessaires à la croissance de cellules, une combinaison de b-FGF, SCF et LIF. En outre, la présence dans le milieu d'un anticorps monoclonal neutralisant l'activité de différenciation exercée par l'acide rétinoïque augmente le nombre de cellules souches embryogènes totipotentes.

La présence d'un tapis de cellules nourricières favorise la croissance de cellules ES aviaires. Divers types de cellules connues de l'homme du métier peuvent être utilisées ; on peut citer en particulier des cellules telles que les cellules STO, traitées à la mitomycine ou irradiées, les cellules BRL-3A, les cellules LMH, les cellules QT6 et cellules QT6 modifiées telles que les cellules QT6 Isolde, les cellules différenciées établies en lignée à partir des cultures de cellules souches embryonnaires induites à différencier.

Les cellules STO sont des fibroblastes d'embryons de souris (catalogue ATCC) ; les cellules BRL-3A (catalogue ATCC) sont des cellules de foie de "Buffalo rat liver". Les cellules QT6 (catalogue ATCC) et cellules QT6 modifiées telles que les cellules QT6 Isolde sont des fibroblastes de caille (Cosset et col., 1990, J. Virol. 64, 10170-1078) et les cellules LMH proviennent de carcinome de foie de poulet (Kawagucchi et col., 1987, Cancer Res., 47, 4460-4464).

Le milieu de culture contient en outre différents éléments nutritifs essentiels et des antibiotiques.

Un milieu de culture particulièrement adapté à la présente invention possède la composition suivante :

| BHK-21 | |
|---|---|
| Sérum foetal de bovin | 10% |
| Sérum de poulet | 2% |
| Conalbumine | 20 ng/ml |
| Acides aminés non essentiels | 1% |
| Pyruvate de sodium | 1 mM |
| Nucléosides stock | 1% |
| Hepes (1M) | 10 mM |
| β-mercaptoéthanol | 0,2 mM |
| Penicilline | 100 U/ml |
| Streptomycine | 100 µg/ml |
| Gentamycine | 10 ng/ml |

| Additifs: | |
|---|---|
| | Final |
| bFGF | de 1 à 20 ng/ml |
| a-SCF | de 0,5% à 2% vol SN de COS/vol |
| IGF-1 | de 5 à 50 ng/ml |
| LIF | de 1000 à 5000 U/ml de forme purifiée soit environ de 0,1% à 2% vol/vol de surnageant de culture de cellules COS transfectées |
| IL-6 | de 5 à 50 ng/ml (environ de 0,1% à 2% vol/vol de surnageant de culture de cellules COS transfectées) |
| IL-11 | de 5 à 50 ng/ml (environ de 0,1% à 2% vol/vol de surnageant de culture de cellules COS transfectées) |

De manière avantageuse la concentration en bFGF est supérieure à 5 ng/ml et la concentration en IGF-1 est supérieure à 10 ng/ml. avec le stock de nucléosides constitué du mélange :

| | |
|---|---|
| adénosine | 80 mg |
| guanosine | 85 mg |
| cytidine | 73 mg |
| uridine | 73 mg |
| thymidine | 24 mg |
| H₂O | 100 ml |

et SN de Cos représentant un surnageant de culture de cellules COS-7 transfectées en expression transitoire avec un vecteur d'expression du cDNA du facteur considéré,
et convient à la culture de cellules embryonnaires totipotentes d'oiseau.

Le milieu BHK21 (ou milieu MEM) est un milieu de culture qui a été décrit notamment par Mc Pherson, I., et Stoker (1962, Virology 16, 147).

Hepes est de l'hydroxy-éthyl-pipérazine-éthane-sulfonate.

Selon un autre aspect, l'invention a pour objet un procédé de culture de cellules embryonnaires totipotentes aviaires (ou cellules ES aviaires), caractérisé en ce que :
a) on met en suspension des cellules provenant de disques blastodermiques d'oeufs fécondés dans un milieu de culture pour cellules aviaires comprenant en outre au moins un composé choisi parmi les cytokines, les facteurs de croissance des fibroblastes, les facteurs de croissance analogue de l'insuline, les facteurs de croissance des cellules souches, ledit milieu étant dépourvu d'acide rétinoïque actif pour action des anticorps anti-acide rétinoïque (ARMA) présents dans ledit milieu
b) on ensemence un tapis de cellules nourricières ou une boite de culture gélatinée avec la suspension obtenue à l'issue de l'étape a),
c) on met les cellules à incuber
d) les cellules en culture sont prélevées et purifiées afin de récupérer des cellules ES d'oiseau.

De préférence, entre les étapes c) et d) on effectue une ou plusieurs additions échelonnées dans le temps, de milieu neuf identique à celui utilisé dans l'étape a).

Dans un de ses modes de mise en oeuvre, au cours de l'étape c), on effectue un réensemencement du milieu par une suspension de cellules identique à la suspension préparée à l'étape a).

Le milieu de l'étape a) contient de préférence les éléments suivants: b-FGF, a-SCF, IGF-1, LIF, IL-11, IL-6 et anticorps anti-acide rétinoïque. Selon l'un des aspects de l'invention, il contient en outre les composés suivants :
. Sérum foetal de bovin
. Sérum de poulet
. Conalbumine
. Acides aminés non essentiels
. Pyruvate de sodium
. Stock de nucléosides
. Hepes (1M)
. β-mercaptoéthanol
. Penicilline
. Streptomycine
. Gentamycine
avec le stock de nucléosides, constitué du mélange :
adénosine, guanosine, cytidine, uridine et thymidine en solution aqueuse.

De manière facultative, au cours du procédé selon l'invention, on effectue entre les étapes c) et d) l'addition de milieu neuf au 3ème jour puis le milieu est changé tous les jours jusqu'au prochain repiquage.

L'étape d) peut notamment être effectuée par traitement enzymatique, lavage dans un milieu ne contenant pas de facteur de croissance et centrifugation.

On peut recueillir directement les cultures primaires de cellules, qui seront ensuite congelées, ou bien réaliser des cultures secondaires successives à partir des cellules de la culture primaire. Dans ce cas, à l'issue de l'étape d), on effectue une étape c) dans laquelle, les cellules ES sont réensemencées sur un tapis de cellules nourricières, ou sur boîtes gélatinées, de manière à obtenir une culture secondaire.

Les étapes d) et e) peuvent être répétées plusieurs fois pour avoir des cultures tertiaires et successives.

Le tapis de cellules nourricières peut être constitué de différents types de cellules décrits précédemment, notamment de cellules STO mitomycinées ou irradiées.

Un autre des objets de l'invention est une culture de cellules ES d'oiseau, ou des cellules ES aviaires, susceptibles d'être obtenues par le procédé défini ci-dessus. Une cellule embryonnaire totipotente aviaire modifiée peut être obtenue par intégration du gène codant pour une protéine hétérologue dans le génome d'une cellule ES-aviaire en culture.

Enfin, un procédé de production d'une protéine recombinante, caractérisé en ce qu'on intègre le gène codant pour ladite protéine dans le génome d'une cellule embryonnaire totipotente aviaire en culture est également compris dans l'invention.

La Demanderesse a mis au point un milieu de culture et des conditions de culture in vitro permettant de maintenir en culture des cellules d'oiseau qui présentent des propriétés morphologiques, cinétiques ct histochimiqucs rappelant celles des cellules embryonnaires totipotentes. Ces observations ont été effectuées aussi bien avec des cellules dérivant de disques blastodermiques de caille que de poulet. La croissance de ces cellules en culture in vitro est rendue possible par la mise au point d'un milieu original spécialement adapté à la culture de cellules embryonnaires d'oiseau. On sait que la présence, le maintien et la propagation de cellules totipotentes en culture permettent leur injection dans des embryons receveurs. La contribution à la morphogenèse des tissus somatiques et germinaux chez les animaux receveurs grâce à un caractère totipotent, peut conduire à l'obtention d'animaux transgèniques.

Les exemples qui suivent sont destinés à illustrer l'invention sans aucunement en limiter la portée. Dans ces exemples, on se référera aux figures suivantes :
**Figure 1:** Effet des combinaisons de facteurs
   - blastodermes de caille, 0,75 bl/ml
   - fond de gélatine
   - culture de 3 j
**Figure 2:** effet de l'anticorps anti-acide rétinoïque (ARMA)
   - blastodermes de caille, 0,75 bl/ml
   - fond avec ou sans gélatine
   - culture de 4 j
**Figure 3:** comparaison de différentes cytokines
   - blastodermes de caille, 2 bl/ml
   - fond avec gélatine
   - culture de 2 + 3 j
**Figure 4**: Comparaison d'un ensemencement sur gélatine et sur tapis de cellules traitées à la mitomycine C en présence de différentes cytokines appartenant toutes à la même famille.
   4A:
      - blastodermes de caille, 1 + 1,5 bl/ml
      - fond avec gélatine
      - culture de 3 + 4 j
   4B:
      - blastodermes de caille, 1 + 1,5 bl/ml
      - fond avec cellules STO
      - culture de 3 + 4 j
**Figure 5**: Activité phosphatase alcaline et reconnaissance par ECMA-7
   - blastodermes de caille, 1,5 bl/ml
   - fond avec cellules STO
   - culture de 2 + 3 j
**Figure 6:** Activité phosphatase alcaline et reconnaissance par NC-1
   - blastodermes de caille, 1,5 bl/ml
   - fond avec cellules STO
   - culture de 2 + 3 j
**Figure 7 :** Animaux chimères obtenus par injection in ovo dans des embryons de cellules maintenues en culture. Cellules injectées après 8 ou 10 jours de culture.

### Matériel et methodes

### Préparation des cellules

Les oeufs de poules fraichement pondus, non incubés, correspondent au stade X de développement (Eyal Giladi and Kovak, 1976) ; les oeufs de caille " C. coturnix japonica" sont également utilisés dès la ponte et non incubés.

Le disque blastodermique (3-4 mm de diamètre pour la poule, 2-2,5 mm pour la caille) est prelevé à l'aide d'une pipette pasteur dans du milieu complet sans facteurs. Les cellules sont centrifugées à 200 g, lavées deux fois dans du milieu afin d'éliminer le maximum de vitellus contaminant, resuspendues à raison de 2 disques pour 1 ml de milieu et dissociées mécaniquement par passage dans une aiguille de 23 G. Les facteurs sont alors ajoutés.

La suspension cellulaire est déposée:
- soit sur boites ou puits (Costar) préalablement gélatinés (0,2 % gélatine, 1 h à t° ambiante),
- soit sur un tapis de cellules STO préalablement traitées à la mitomycine C (90 min, 37°c, 5 µg/ml) et ré-ensemencées à raison de 10⁵ cellules / cm ²,
- soit sur un tapis de cellules Isolde préalablement traitées à la mitomycine C (90 min, 37°c, 5 µg/ml) et ré-ensemmencées à raison de 10⁵ cellules / cm ².

| Milieu de culture STO : | |
|---|---|
| | final |
| DMEM | |
| Sérum foetal Bovin | 10% |
| Penicilline | 100 U/ml |
| Streptomycine | 100 µg/ml |
| L-Glutamine | 2 mM |

| Milieu de culture Isolde : | |
|---|---|
| | final |
| DMEM | |
| Sérum foetal Bovin | 8 % |
| Sérum de poulet | 2 % |
| Penicilline | 100 U/ml |
| Streptomycine | 100 µg/ml |
| G418 | 100 µg/ml |
| Hygromycine | 50 µg/ml |
| Phléomycine | 50 µg/ml |
| TBP (bouillon tryptose phosphate) | 10% |

Les drogues de sélection sont ajoutées en entretien mais enlevées deux jours avant le traitement à la mitomycine C.

Dans tous les cas, un second ensemencement est réalisé dans les mêmes conditions après deux jours de culture.

### Cultures

Les cultures sont incubées à 37 °C ou à 41° C, dans une atmosphère controlée en CO₂ (7,5 %) et leur évolution est suivie au microscope en contraste de phase. Une addition partielle (50 %) de milieu neuf avec les facteurs est réalisée le 3 ^{ème} jour de culture, puis le milieu est changé tous les jours. A chaque moment, les cellules en croissance peuvent être soit fixées pour étude, soit prélevées pour être ré-ensemencées en culture secondaire ou supérieure, sur tapis de cellules STO mitomycinées irradiées ou sur boîtes gélatinées.

En cas de fixation, les cellules sont lavées en Tris-Glucose deux fois, puis fixées in situ 15 min dans une solution de paraformaldéhyde 4 % à froid (0-4 °C). Aprés plusieurs lavages au PBS, différentes colorations peuvent être réalisées selon l'un des protocoles suivants :
* détection de l'activité phosphatase alcaline endogène,

| | | |
|---|---|---|
| tampon de réaction | NaCl | 100 mM |
| | Tris HCl pH 9.5 | 100 mM |
| | MgCl₂ | 5 mM |
| | NBT | 1 mg/ml |
| | BCIP | 0,1 m/ml |
| | H₂O | |

(temps de lecture, de 5 à 30 min, 37°c)
** détection de l'activité de β-galactosidase exogène

| | | |
|---|---|---|
| tampon de réaction | Ferricyanure de K | 5 mM |
| | Ferrocyanure de K | 5 mM |
| | MgCl₂ | 5 mM |
| | X-gal | 1 mg/ml |
| | PBS | |

(temps de lecture, de 1 à 2heures , 37°c)
*** détection par immunocytochimie de la présence d'épitopes spécifiques (réaction à 4°c)

| | |
|---|---|
| blocage en tampon PBS - BSA (1 mg/ml) | |
| lavage en PBS - BSA | |
| anticorps primaire | 1/ 10^{ème} ou 1/50^{éme} |
| anticorps secondaire fluorescent | 1/50^{ème} |

la détection est réalisée sous microscope inversé à fluorescence.

### Repiquage

En cas de passage en culture secondaire ou successive, les cellules sont lavées en Tris-Glucose deux fois, puis incubées 10-30 min dans une solution enzymatique. On peut utiliser une solution de collagénase-dispase (1 mg/ml soit 1 U/ml final) à laquelle une solution de hyaluronidase (1 mg/ml final soit 1 U/ml) peut être ajoutée ; on peut également utiliser une solution de pronase à 0,25 mg/ml final. Les cellules ou les petits amas de cellules ainsi isolés enzymatiquement sont lavées en milieu ESA, resuspendus, déposés sur un coussin de milieu de séparation de lymphocyte de densité (d= 1,077-1,080) et centrifugés 20 min à t° ambiante à 800 g afin de débarasser les cellules non différenciées de blatodermes des cellules du tapis, des débris divers et des restes de vitellus contaminants. L'interface est alors prélevée, lavée deux fois en milieu ESA. Le culot cellulaire obtenu est resuspendu et dissocié légérement mécaniquement avant d'être ensemencé sur un nouveau tapis de cellules nourricières, comme précédemment décrit. L'équivalent de 6 disques blastodermiques initiaux est ré-ensemencé dans 2 ml. Cette étape de gradient n'est parfois pas nécessaire lors des passages successifs, en fonction de la très grande homogénéité des cultures obtenues.

Les cellules dissociées peuvent être déposées sur un gradient multicouche de Percoll et centrifugées dans les mêmes conditions. Les interfaces sont alors prélevées, lavées dans un milieu ESA et les cellules les plus immatures des interfaces supérieures réensemencées, ou injectées dans des embryons receveurs.

### Congélation

A l'issue de la culture primaire ou successive, les cellules récupérées de gradient peuvent être congelées dans un mélange constitué de 40 % FBS, 50 % milieu ESA et 10 % DMSO. Les cellules équivalant à 24 blastodisques initiaux sont reprises dans 0,5 ml de milieu ESA, resuspendues et 0,4 ml de sérum est ajouté 0,1 ml de DMSO est alors ajouté très lentement. La suspension de congélation est répartie dans des tubes à congélation (0,5 ml/tube) et congelée lentement à -80 °c avant d'être transférée dans l'azote liquide.

### Resultats

Un milieu de base appelé milieu "ESA" pour "Embryonic Stem cells Avian" dérivant d'un milieu utilisé pour les cellules ES murines a été préparé. Il présente la composition suivante :

| Milieu "ESA": | |
|---|---|
| | final |
| BHK-21 | |
| Foetal Bovine Serum | 10% |
| Serum de poulet | 2 % |
| Conalbumine | 20 ng/ml |
| Acide Aminés non essentiels | 1 % |
| Pyruvate de sodium | 1 mM |
| Stock de nucléosides | 1 % |
| Hepes (1M) | 10 mM |
| β-mercaptoethanol | 0.2 mM |
| Penicilline | - 100 U/ml |
| Streptomycine | 100 µg/ml |
| Gentamycine | 10 ng/ml |

A ce milieu de base "ESA", des facteurs de croissance ont été ajoutés afin de comparer leur contribution respective à la formation de colonies présentant un caractère morphologique et biochimique intéréssant. Leurs concentrations sont indiquées ci-après :

| Additifs: | | |
|---|---|---|
| | stock | final |
| bFGF | 10 µg/ml | 10 ng/ml |
| a-SCF | SN de Cos trans* | 1 % vol/vol |
| IGF-1 | 10 µg/ml | 20 ng/ml |
| LIF | SN de Cos trans* | 1 % vol/vol |
| IL-1 1 | 10 µg/ml | 10 ng/ml |
| IL-6 | 10 µg/ml | 10 ng/ml |
| ARMA | 10 mg/ml | 1 µg/ml |
| OSM | 20 µg/ml | 20 ng/ml |
| CNTF | 20 µg/ml | 20 ng/ml |

| stock de nucléosides | | |
|---|---|---|
| | adénosine | 80 mg |
| | guanosine | 85 mg |
| | cytidine | 73 mg |
| | uridine | 73 mg |
| | thymidine | 24 mg |
| | H₂O | 100 ml |

| | | |
|---|---|---|
| * surnageant de culture de cellules COS-7 transfectées en expression transitoire avec un vecteur d'expression du cDNA du facteur considéré. | | |

Le premier critère utilisé pour évaluer l'effet de ces facteurs et des modifications apportées au milieu a été la détection par coloration biochimique de l'activité phosphatase alcaline endogène qui semble spécifique d'un certain nombre de cellules telles que les cellules ES totipotentes, les cellules précurseurs dérivées de la lignée germinale et certaines cellules différenciées, facilement identifiables à leur morphologie épithélioïde.

Les cellules des disques blastodermiques sont ensemencées en milieu ESA en présence de différentes combinaisons de facteurs. Après 3 j de culture, les cellules sont fixées, colorées et les colonies positives pour l'activité phosphatase alcaline (AP+) sont dénombrées.

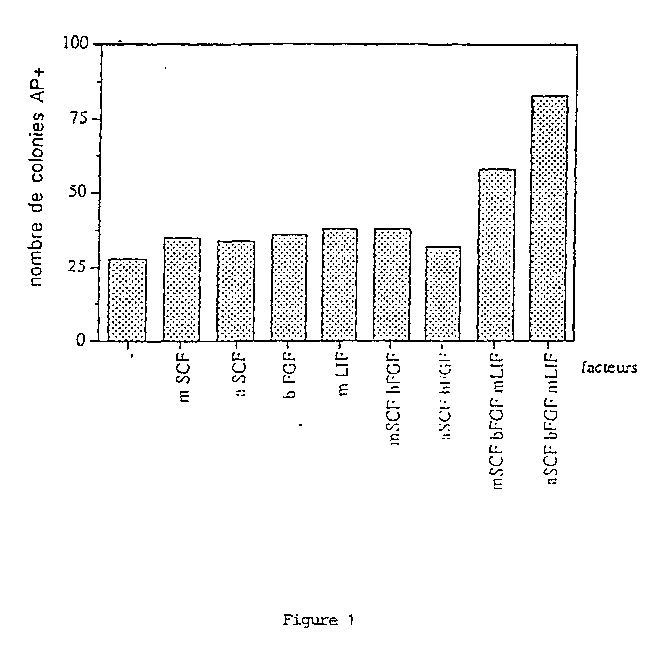

L'effet des différentes combinaisons de facteurs est représenté sur la figure 1.

### Conclusion

Parmi les facteurs testés, la combinaison du SCF (Stem Cell Factor d'origine murine -mSCF- ou aviaire -aSCF-), du b-FGF (basic Fibroblast Growth Factor) et du LIF (Leukemia Inhibitory Factor) donne le meilleur nombre de colonies positives pour l'activité phosphatase alcaline dans les cultures avec un accroissement de 2-3 fois par rapport à la présence de chaque facteur ajouté individuellement ou deux par deux et par rapport au fond, constitué en majorité de cellules faiblement positives et présentant une morphologie épithélioïde différenciée.

### II) Effet de l'anticorps anti-acide rétinoïque (ARMA)

Les cellules sont ensemencées soit sur boites non traitées, soit traitées à la gélatine dans du milieu ESA complet avec facteurs de croissance aSCF (avian Stem Cell Factor), bFGF (basic- Fibroblast Growth Factor), IGF-1 (Insulin-like Growth Factor-1) et LIF (Leukemia Inhibitory Factor). L'anticorps ARMA est ajouté à raison de 1 µg/ml final. Les cellules et colonies positives pour l'activité phosphatase alcaline (AP +) sont dénombrées après 4 j de culture.

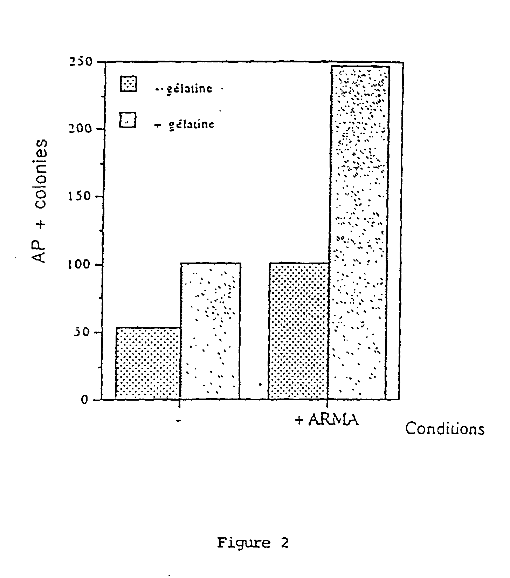

Les résultats sont représentés sur la figure 2.

Comparativement aux différents moyens décrits comme l'utilisation de résine ou de charbon, et testés pour essayer de controler le niveau d'acide rétinoïque dans le milieu, l'addition de l'anticorps anti acide rétinoïque dans le milieu donne les meilleurs résultats quant à la qualité et la quantité des colonies présentes dans les cultures

### Conclusion

L'addition de l'anticorps anti-acide rétinoïque dans le milieu de culture accroit de façon notable la présence et /ou le maintien des colonies à activité phosphatase alcaline.

### III) Effet des cytokines

Nous avons voulu vérifier si le LIF ou d'autres cylokines de la même famille pouvait induire la prolifération des cellules ES chez l'oiseau.

Les cellules sont ensemencées en milieu ESA complet avec facteurs de croissance aSCF (avian Stem Cell Factor), bFGF (basic- Fibroblast Growth Factor), IGF-1 (Insulin-like Growth Factor-1) en présence d'ARMA (1 µg/ml) et après addition ou non de différentes cytokines de la même famille LIF (Leukemia Inhibitory Factor), IL-11 (Interleukine 11) et IL-6 (Interleukine 6). Afin d'accroître l'adhésion et la formation de colonies phosphatase alcaline positives ainsi que leur taille, un second ensemencement a lieu 2 jours après le premier. La fixation, coloration et lecture des colonies a eu lieu 3 jours après le second ensemencement.

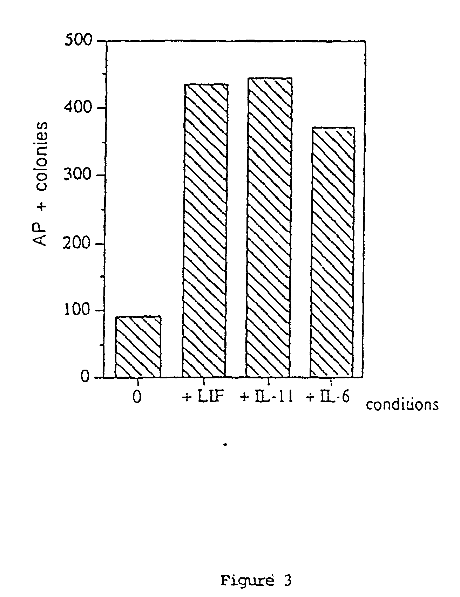

La comparaison de l'effet des différentes cytokines est représentée sur la figure 3.

### Conclusion

Le rôle des cytokines LIF, IL-11 et IL-6 semble particulièrement prononcé et pratiquement équivalent dans l'obtention de colonies positives pour l'activité phosphatase alcaline.

### IV) Rôle d'un tapis de cellules nourricières

Chez la souris, la croissance de certaines cellules ES requiert la présence d'un tapis de cellules nourricières. L'effet de ces cellules sur les cellules d'embryons d'oiseau a été testé

Les cellules sont ensemencées dans un milieu ESA complet avec facteurs de croissance aSCF (avian Stem Cell Factor), bFGF (basic-Fibroblast Growth Factor), IGF-1 (Insulin-like Growth Factor-1) et anticorps ARMA (1 µg/ml) comparativement soit sur un fond de gélatine soit sur un tapis de cellules STO traitées à la mitomycine C comme indiqué dans le paragraphe Matériel et Méthodes. Après trois jours de culture, un nouvel ensemencement est ajouté à la culture. Les cytokines CNTF (Ciliary Neuro-Trophic Factor), OSM (Oncostatin M), LIF (Leukemia Inhibitory Factor), IL-11 (Interleukine 11) et IL-6 (Interleukine 6) sont ajoutées dans le milieu aux concentrations indiquées précédemment.

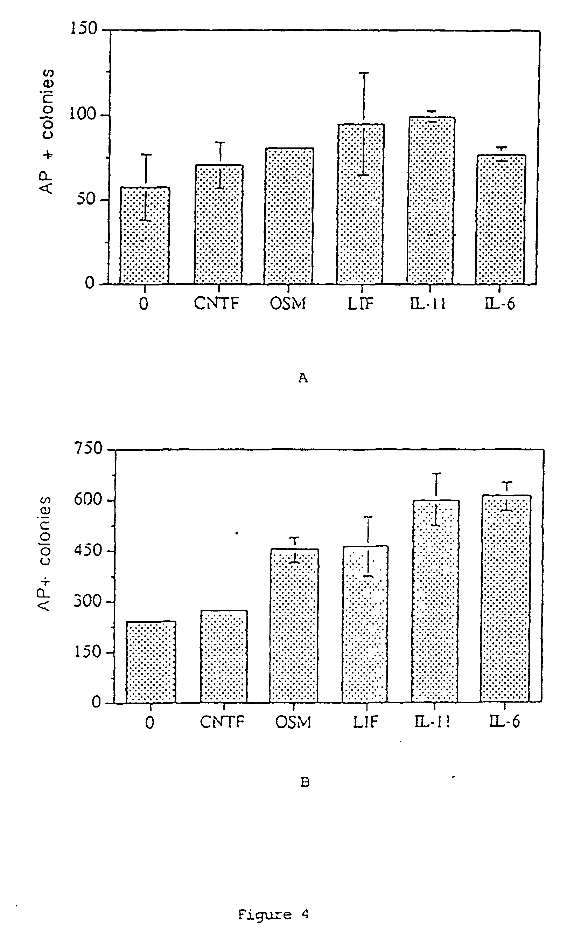

La figure 4A représente la croissance de cellules sur gélatine en présence de différentes cytokines. La figure 4B représente la croissance de cellules sur un tapis de cellules nourricières en présence des mêmes cytokines.

### Conclusion

Le nombre de colonies dérivant des cellules de blastoderme et présentant une activité phophatase alcaline est très nettemment accru en présence d'un tapis de cellules nourricières (environ 4-5 fois) avec un maintien entre les deux systèmes des même sensibilités vis à vis des cytokines ajoutées dans le milieu. Les cytokines LIF, IL-11 et II-6 présentent les meilleurs résultats de stimulation de croissance. Dans des résultats préliminaires, il apparaît de plus que la combinaison de ces 3 cytokines dans le milieu complet ESA avec facteurs produisent des effets cumulatifs très prometteurs quant au maintien et à la prolifération des coloniés tant avec des cellules dérivées de disques blastodermiques de caille que de poulet.

### V) Caractéristiques immunocytochimiques

Des études de réactivité par rapport à différents anticorps ont été réalisées. Les anticorps ECMA-7, SSEA-1 et SSEA-3, spécifiques d'un état de totipotence des cellules ES murines sont capables de reconnaitre des épitopes dans les populations de cellules aviaires, maintenues dans les cultures. Pour illustrer ces reconnaissances par les anticorps, des doubles marquages activité phosphatase alcaline et anticorps démontrent que toutes les cellules ou les massifs de cellules reconnues par ECMA-7 présentent une activité phosphatase alcaline. Cette propriété a été observée avec tous les anticorps utilisés à des degrés divers.

Les colonies de cellules phosphatase alcaline positives sont pour environ 20 % d'entre elles marquées par l'anticorps ECMA-7. Cette reconnaissance suggère la présence dans ces massifs et dans ces seules conditions de culture de cellules à caractère "ES". Néanmoins, une hétérogénéité dans les massifs phosphatase alcaline positifs suppose des degrés variables dans l'intensité du caractère "ES".

Cette hétérogénéité de distribution a été observée sur des cultures primaires. Après repiquages, la proportion de cellules positives, notamment pour ECMA-7, mais également pour SSEA-1 et EMA-1, tend à s'accroître de façon très importante pour obtenir des cultures très homogènes.

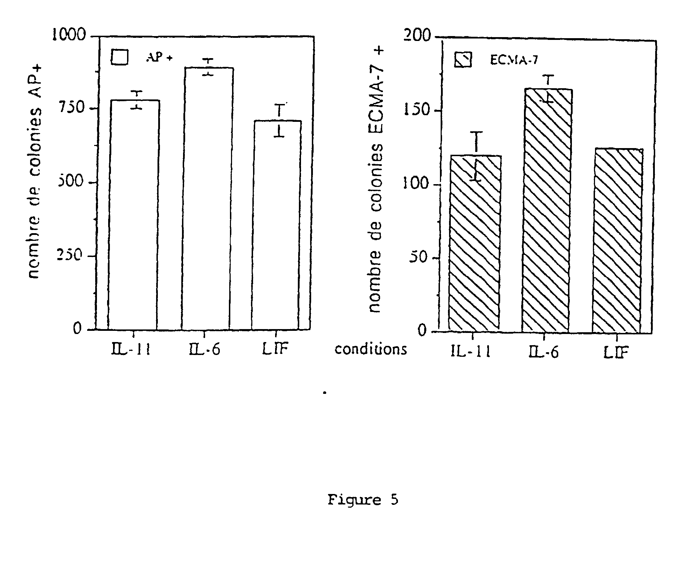

La figure 5 montre respectivement l'activité phosphatase alcaline et la reconnaissance par l'anticorps ECMA-7 de colonies de cellules issues de la culture de blastodermes de caille en présence de différentes cytokines.

Les anticorps SSEA-1, SSEA-3, également utilisés sur des cellules ES murines reconnaissent également des cellules aviaires dans les massifs phosphatase alcaline positifs.

Les anticorps NC-1, HNK-1 dirigés respectivement des épitopes de cellules de crêtes neurales et de cellules "human natural killer" reconnaissent en fait les mêmes épitopes et ont été montrés comme reconnaissant certaines cellules immatures du disque blastodermique de poulet. Dans notre système, ces deux anticorps reconnaissent là encore des cellules dans des massifs à activité phosphatase alcaline.

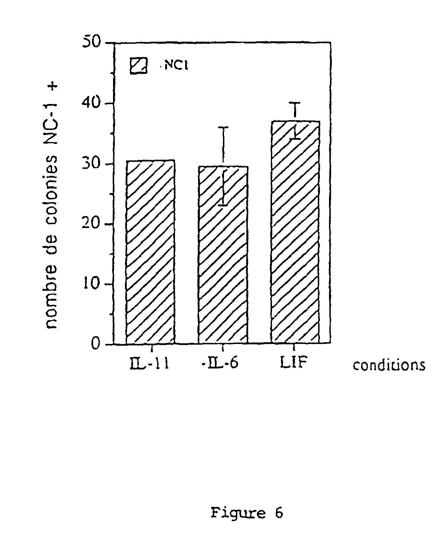

Les résultats avec NC-1 sont représentés sur la figure 6.

Les cellules sont ensemencées dans un milieu ESA complet avec facteurs de croissance aSCF (avian Stem Cell Factor), bFGF (basic-Fibroblast Growth Factor), IGF-1 (Insulin-like Growth Factor-1) et anticorps ARMA (1 µg/ml) sur un tapis de cellules STO traitées à la mitomycine C comme indiqué dans le paragraphe Matériel et Méthodes. Après deux jours de culture, un nouvel ensemencement est ajouté à la culture. Les cytokines LIF (Leukemia Inhibitory Factor), IL-11 (Interleukine 11) et IL-6 (Interleukine 6) sont ajoutées dans le milieu aux concentrations indiquées précédemment. La double coloration, activité phosphatase alcaline et détection des épitopes par anticorps est réalisée suivant les protocoles présentés précédemment.

Par ailleurs, l'anticorps EMA-1 (Hahnel and Eddy, 1986) initialement dirigé contre des épitopes présents sur les cellules primordiales de la lignée germinale murine a été utilisé contre ces mêmes cellules chez le poulet. En testant cet anticorps dans notre système de culture, nous pouvons démontrer que EMA-1 reconnait des cellules et colonies de cellules présentant toutes une activité phosphatase alcaline. Il a été par ailleurs vérifié que cet anticorps EMA-1 ne reconnaissait les cellules ES murines que dans leur état de totipotence non différenciée.

Les anticorps ont été testés soit sur des cultures non différenciées obtenues telles que décrites dans Matériel et Méthodes soit sur des cultures qui ont été traitées avec un excès d'acide rétinoïque ajouté à la culture (10⁻⁶ M) pendant au moins 48 heures. Le tableau ci-après indique l'état de reconnaissance par les différents anticorps utilisés.

| Anticorps monoclonal | Non differenciées | Differenciées |
|---|---|---|
| | | |
| ECMA-7 | +++++ | - |
| SSEA-1 1 | +++++ | - |
| SSEA-3 | +++ | - |
| TEC-O1 | +++++ | - |
| TEC-02 | + | +++ |
| TEC-03 | ++ | ++ |
| EMA-1 | ++++ | + |
| EMA-6 | +++ | + |
| TROMA-1 | - | ++++ |
| NC-1 | ++++ | + |
| HNK-1 | ++++ | + |
| NC-1 et HNK-1 reconnaissent les mêmes épitopes (Tucker et al, 1984) SSEA-1 et TEC 01 reconnaissent les mêmes épitopes | | |

Il apparaît que l'expression de ECMA-7 (Kemler et al. (1981)) est la plus importante, suggérant une véritable nature de cellules ES, que TEC-01 (Draber et al. (1987b)) et SSEA-1 (Solter and Knowles (1978)) reconnaissent les mêmes épitopes sur des cellules non différenciées exclusivement. A l'inverse, l'augmentation d'expression de TEC-02 (Draber et al. (1987a)) peut dans ce sens indiquer un état de différenciation induit ou spontané. L'achèvement de cette perte de nature ES est caractérisée par la forte expression de TROMA-1 (Brulet et al. (1980)), présent sur les seules cellules différenciées. L'ensemble de ces anticorps permet donc d'avoir une idée sur l'état de différenciation d'une culture. Des anticorps comme TEC-03 (Draber et al. (1987a)) apparaissent comme relativement indifférents à l'état prononcé de différenciation.

Il est par ailleurs à souligner que jusqu'à présent ni ECMA-7, ni SSEA-1, SSEA-3, TEC-01, TEC-02, TEC-03, TROMA-1 n'ont fait l'objet de publication démontrant la réactivité sur des coupes, des cellules ou tout matériel d'origine aviaire.

### Conclusion

Parmi les anticorps testés, certains comme ECMA-7 (Kemler et al. (1981)), SSEA-1 (Solter and Knowles (1978)), SSEA-3 (Shevinsky et al. (1982)) sont caractéristiques des cellules "ES "murines. Ces anticorps reconnaissent des cellules donc potentiellement totipotentes dans les cultures aviaires. Les mêmes observations ayant été obtenues soit avec des cultures de caille soit de poule. D'autres anticorps comme EMA-1 (Hahnel and Eddy (1986)), NC-1 et HNK-1 (Obo and Balch (1981)) sont connus pour reconnaitre des épitopes aviaires (et murin pour EMA-1, Urven et al, (1988)) de cellules très indifférenciées et donc aussi susceptibles de reconnaitre un profil de cellules souches aviaires.

### VI) Repiquage des cellules

Les cellules de disques blastodermiques de caille ou de poulet sont ensemencées sur tapis de cellules nourricières STO. Après différents jours de culture, les cellules sont repiquées sur tapis de cellules STO comme décrit en Matériel et méthodes. La détection de cellules et massifs positifs à la fois pour l'activité de phosphatase alcaline et pour la colocalisation d'un marquage par ECMA-7 ou NC-1 suggère que les conditions de cultures sont définies pour maintenir dans les cultures secondaires et tertiaires des cellules à caractère totipotent. Le processus de repiquage assure d'ailleurs dès après le premier passage une homogénéité à l'ensemble de la culture, tant morphologiquement, que par la détection des différents épitopes. Les massifs de cellules deviennent très étendus et homogènes, caractère accru par la grande capacité de ces cellules à se diviser rapidement, contrairement aux cellules différenciées présentes initialement dans la culture primaire. Jusqu'à présent, ces critères d'identification et de caractérisation peuvent être utilisés et détectés pendant au moins 5 semaines après l'ensemencement.

### VII) Injection des cellules dans des embryons receveurs

Les cellules blastodermiques de poulet obtenues en cultures primaires ou après repiquages successifs peuvent être injectées dans des embryons receveurs. Afin de visualiser rapidement une contribution phénotypique des cellules du donneur dans un embryon de poussin receveur, les cellules maintenues en culture proviennent d'une souche pigmentée et les embryons receveurs d'une souche non pigmentée. Les cellules maintenues en cultures sont dissociées et préparées comme décrit dans Matériel et Méthodes selon le même procédé que pour un repiquage. La suspension cellulaire est alors préparée à raison de 1 à 3 x 10⁵ cellules par ml de milieu ESA. L'oeuf fraîchement pondu, non incubé contenant l'embryon reveceur est légèrement irradié entre 5 Gy et 7 Gy. Une petite fenêtre de quelques mm² est réalisée dans la coquille du receveur par meulage. La membrane coquillière est découpée au scalpel et les cellules sont injectées à l'aide d'un capillaire étiré dans la cavité subgerminale du disque blastodermique dans un volume de 1 à 5 µl, ce qui correspond de 100 à 1500 cellules au maximum. La moyenne des cellules injectées est de 500 cellules. La fenêtre est alors recouverte de membranes coquillières et scellée. Un morceau de pansement adhésif est appliqué pour parfaire l'étanchéité et limiter au maximum l'évaporation. Après 4 jours d'incubation dans les conditions optimales, les oeufs sont ouverts et les embryons bien développés sont transférés dans une coquille plus grande et remis en incubation pour finir leur développement de façon satisfaisante.

Un certain nombre d'animaux ont ainsi été obtenus et montrent un taux apparent de chimérisme, phénotypiquement détectable par le marqueur de plumage utilisé et caractéristique de la souche des cellules dérivant de la souche donneur, variant de 5% à 90%. Ce chimérisme peut être obtenu jusqu'à présent indifféremment avec des cellules dérivant de cultures primaires, secondaires, tertiaires. Il est à noter que les pourcentages d'animaux chimères et les taux de chimérisme de ces animaux ne varient pas de façon importante en fonction du temps de culture des cellules injectées. Ceci contribue a souligner la capacité du milieu et au procédé décrit à maintenir des cellules avec un caractère totipotent.

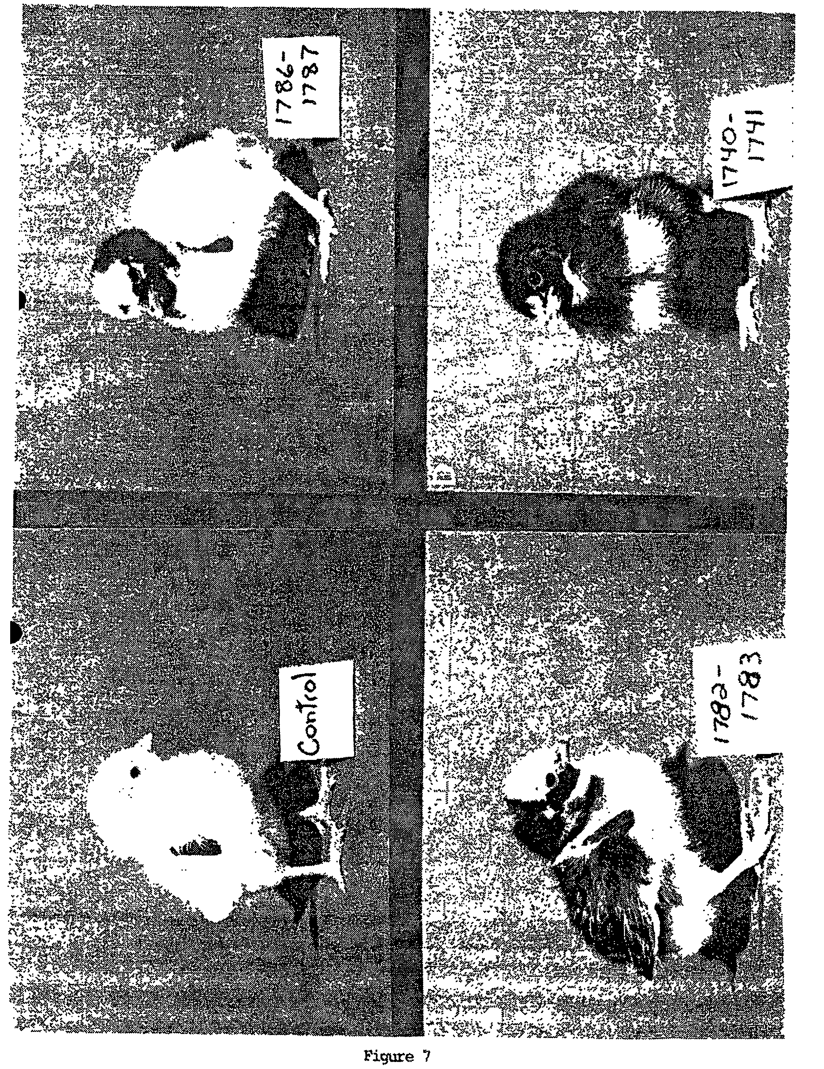

### Exemple : animal controle non injecté (Fig. 7A)

Animal n° 1786-1787 à faible taux de chimérisme (5-10%)
Animal n° 1782-1783 à taux moyen de chimérisme (50%)
Animal n° 1740-1741 à fort taux de chimérisme (90%)

Ces animaux sont présentés sur les figures 7B à 7D.

### REFERENCES

Brulet et al. (1980). Proc. Natl. Acad. Sci. 77, 4113.
Draber et al. (1987a). Cell Differentiation 21, 119.
Draber et al. (1987b). Cell differentiation 21, 227.
Eyal Giladi and Kovak (1976), Developemental Biology, 49, 321-337
Hahnel and Eddy (1986). Gamete Research 15, 25.
Kemler et al. (1981). J. Embryo. Exp. Morph. 64, 45.
Obo and Balch (1981). J. Immunology 127, 1024.
Petitte et al (1990) Development 108, 185-189
Solter and Knowles (1978). Proc. Natl. Acad. Sci. 75, 5565.
Shevinsky et al. (1982). Cell 30, 697.
Tucker et al. (1984). Cell Differentiation 14, 223.
Urven et al. (1988). Development 103, 299.

## Revendications

1. Milieu de culture de cellules embryonnaires totipotentes aviaires du type comportant un milieu de culture pour cellules aviaires, **caractérisé en ce qu'**il comprend au moins un composé choisi parmi les cytokines, les facteurs de croissance des fibroblastes, les facteurs de croissance analogues de l'insuline, les facteurs de croissance des cellules souches et **en ce qu'**il comprend des anticorps anti-acide rétinoïque (ARMA).

2. Milieu de culture selon la revendication 1, **caractérisé en ce que** la cytokine est choisie parmi le LIF (leukemia inhibitory factor), IL-11 (interleukine-11), IL-6 (interleukine-6), CNTF (ciliary neurotrophic factor), oncostatine M (OSM) et leurs mélanges.

3. Milieu de culture selon l'une des revendications 1 à 2, **caractérisé en ce qu'**il contient au moins une cytokine choisie dans le groupe constitué de LIF, IL-11, IL-6 et leurs différents mélanges.

4. Milieu de culture selon l'une des revendications 1 à 3, **caractérisé en ce qu'**il contient un facteur de croissance des fibroblastes qui est le b-FGF (basic fibroblast growth factor).

5. Milieu de culture selon l'une des revendications 1 à 4, **caractérisé en ce qu'**il contient un facteur de croissance analogue de l'insuline qui est l'IGF-1 (insuline-like growth factor-1).

6. Milieu de culture selon l'une des revendications 1 à 5, **caractérisé en ce qu'**il contient un facteur de croissance des cellules souches choisi parmi l'a-SCF (avian stem cell factor) et le M-SCF (murine stem cell factor).

7. Milieu de culture selon l'une des revendications 1 à 6, **caractérisé en ce qu'**il comporte un tapis de cellules nourricières.

8. Milieu de culture selon l'une des revendications 1 à 7, **caractérisé en ce qu'**il contient en outre les composés suivants :
- Sérum foetal bovin
- Sérum de poulet
- Conalbumine
- Acides aminés non essentiels
- Pyruvate de sodium
- Stock de nucléosides
- Hepes(1M)
- β-mercaptoéthanol
- Pénicilline
- Streptomycine
- Gentamycine
avec le stock de nucléosides constitué du mélange :
adénosine, guanosine, cytidine, uridine et thymidine en solution aqueuse.

9. Procédé de culture de cellules embryonnaires totipotentes aviaires (ou cellules ES aviaires), **caractérisé en ce que** :
a) on met en suspension des cellules provenant de disques blastodermiques d'oeufs fécondés dans un milieu de culture pour cellules aviaires comprenant au moins un composé choisi parmi les cytokines, les facteurs de croissance des fibroblastes, les facteurs de croissance analogue de l'insuline, les facteurs de croissance des cellules souches, ledit mélange étant dépourvu d'acide rétinoïque actif pour action des anticorps anti-acide rétinoïque présents dans ledit milieu,
b) on ensemence un tapis de cellules nourricières avec la suspension obtenue à l'issue de l'étape a),
c) on met les cellules à incuber,
d) les cellules en culture sont prélevées et purifiées afin de récupérer des cellules ES d'oiseau.

10. Procédé selon la revendication 9, **caractérisé en ce qu'**entre les étapes c) et d) on effectue une ou plusieurs additions échelonnées dans le temps, de milieu neuf identique à celui utilisé dans l'étape a).

11. Procédé selon l'une des revendications 9 ou 10, **caractérisé en ce que** le milieu de l'étape a) contient les éléments suivants : b-FGF, a-SCF, IGF-1, LIF, TL-11, IL-6 et des anticorps anti-acide rétinoïque.

12. Procédé selon l'une des revendications 9 à 11, **caractérisé en ce que** le milieu de l'étape a) contient en outre les composés suivants :
- Sérum foetal bovin
- Sérum de poulet
- Conalbumine
- Acides aminés non essentiels
- Pyruvate de sodium
- Stock de nucléosides
- Hepes (1M)
- β-mercaptoéthanol
- Penicilline
- Streptomycine
- Gentamycine
avec le stock de nucléosides constitué du mélange :
adénosine, guanosine, cytidine, uridine et thymidine en solution aqueuse.

13. Procédé selon l'une des revendications 10 à 12, **caractérisé en ce qu'**entre les étapes c) et d), on effectue l'addition de milieu neuf au 3ème jour, puis tous les jours.

14. Procédé selon l'une des revendications 9 à 13, **caractérisé en ce que** l'étape d) est effectuée par traitement enzymatique, lavage dans un milieu ne contenant pas de facteur de croissance et centrifugation.

15. Procédé selon l'une des revendications 9 à 14, **caractérisé en ce qu'**à l'issue de l'étape d), on effectue une étape e) dans laquelle les cellules ES sont réensemencées sur un tapis de cellules nourricières en présence de milieu selon la revendication 1, de manière à obtenir une culture secondaire.

16. Procédé selon la revendication 15, **caractérisé en ce que** les étapes d) et e) sont répétées plusieurs fois.

17. Procédé selon l'une des revendications 9 à 16, **caractérisé en ce que** le tapis de cellules nourricières est constitué de cellules STO mitomycinées ou irradiées.

18. Culture in vitro de cellules ES aviaires susceptible d'être obtenue par le procédé selon l'une des revendications 10 à 18, ladite culture comprenant un milieu de culture selon la revendication 1.

19. Culture de cellules ES aviaires selon la revendication 18, **caractérisée en ce qu'**elle comprend la combinaison du SCF, du b-FGF et du LIF, ladite culture présentant 2 à 3 fois plus de colonies positives pour l'activité alcaline phosphatase par rapport au fond constitué en majorité de cellules faiblement positives.

20. Culture de cellules ES aviaires selon la revendication 18, **caractérisée en ce que** les cellules sont modifiées par intégration du gène codant pour une protéine hétérologue.

## Patentansprüche

1. Kulturmedium für embryonale totipotente Vogelzellen der Art, die ein Kulturmedium für Vogelzellen umfasst, **dadurch gekennzeichnet, dass** es mindestens eine Verbindung enthält, die ausgewählt ist aus Cytokinen, Wachstumsfaktoren von Fibroblasten, Wachstumsfaktoren, die zu Insulin analog sind, Wachstumsfaktoren von Stammzellen, und dass es Anti-Retinoesäure-Antikörper (ARMA) enthält.

2. Kulturmedium nach Anspruch 1, **dadurch gekennzeichnet, dass** das Cytokin ausgewählt ist aus LIF (Leukemia Inhibitory Factor), IL-11 (Interleukin-11), IL-6 (Interleukin-6), CNTF (Ciliary Neurotrophic Factor), Oncostatin M (OSM) und deren Mischungen.

3. Kulturmedium nach einem der Ansprüche 1 bis 2, **dadurch gekennzeichnet, dass** es mindestens ein Cytokin enthält, das ausgewählt ist aus der Gruppe bestehend aus LIF, IL-11, IL-6 und deren verschiedenen Mischungen.

4. Kulturmedium nach einem der Ansprüche 1 bis 3, **dadurch gekennzeichnet, dass** es einen Wachstumsfaktor von Fibroblasten enthält, bei dem es sich um b-FGF (Basic Fibroblast Growth Factor) handelt.

5. Kulturmedium nach einem der Ansprüche 1 bis 4, **dadurch gekennzeichnet, dass** es einen Wachstumsfaktor enthält, der analog zu Insulin ist, bei dem es sich um IGF-1 (Insuline-like Growth Factor-1) handelt.

6. Kulturmedium nach einem der Ansprüche 1 bis 5, **dadurch gekennzeichnet, dass** es einen Wachstumsfaktor von Stammzellen enthält, der ausgewählt sind aus a-SCF (Avian Stem Cell Factor) und M-SCF (Murine Stem Cell Factor).

7. Kulturmedium nach einem der Ansprüche 1 bis 6, **dadurch gekennzeichnet, dass** es einen Teppich von Nährzellen umfasst.

8. Kulturmedium nach einem der Ansprüche 1 bis 7, **dadurch gekennzeichnet, dass** es darüber hinaus die folgenden Verbindungen enthält:
- fötales Rinderserum
- Hühnchenserum
- Conalbumin
- nicht-essentielle Aminosäuren
- Natriumpyruvat
- Vorrat an Nukleosiden
- Hepes (1M)
- β-Mercaptoethanol
- Penicillin
- Streptomycin
- Gentamycin
wobei der Vorrat an Nukleosiden aus der Mischung:
Adenosin, Guanosin, Cytidin, Uridin und Thymidin in wässriger Lösung besteht.

9. Verfahren zur Kultur von embryonalen totipotenten Vogelzellen (oder Vogel-ES-Zellen), **dadurch gekennzeichnet, dass**:
a) man Zellen, die von blastodermischen Scheiben von befruchteten Eiern abstammen, in einem Kulturmedium für Vogelzellen suspendiert, welches mindestens eine Verbindung enthält, die ausgewählt ist aus Cytokinen, Wachstumsfaktoren von Fibroblasten, Wachstumsfaktoren, die analog zu Insulin sind, Wachstumsfaktoren von Stammzellen, wobei die Mischung für die Wirkung von Anti-Retinoesäure-Antikörpern, die in dem Medium anwesend sind, frei von Retinoesäure ist,
b) man einen Teppich von Nährzellen mit der am Ende von Schritt a) erhaltenen Suspension beimpft,
c) man die Zellen inkubiert,
d) die Zellen in der Kultur geerntet und gereinigt werden, um Vogel-ES-Zellen zu gewinnen.

10. Verfahren nach Anspruch 9, **dadurch gekennzeichnet, dass** man zwischen den Schritten c) und d) eine oder mehrere zeitlich gestaffelte Zugaben von neuem Medium bewirkt, welches identisch ist mit dem im Schritt a) verwendeten.

11. Verfahren nach einem der Ansprüche 9 oder 10, **dadurch gekennzeichnet, dass** das Medium des Schritts a) die folgenden Elemente enthält: b-FGF, a-SCF, IGF-1, LIF, IL-11, IL-6 und Anti-Retinoesäure-Antikörper.

12. Verfahren nach einem der Ansprüche 9 bis 11, **dadurch gekennzeichnet, dass** das Medium des Schritts a) darüber hinaus die folgenden Verbindungen enthält:
- fötales Rinderserum
- Hühnchenserum
- Conalbumin
- nicht-essentielle Aminosäuren
- Natriumpyruvat
- Vorrat an Nukleosiden
- Hepes (1M)
- β-Mercaptoethanol
- Penicillin
- Streptomycin
- Gentamycin
wobei der Vorrat an Nukleosiden aus der Mischung:
Adenosin, Guanosin, Cytidin, Uridin und Thymidin in wässriger Lösung besteht.

13. Verfahren nach einem der Ansprüche 10 bis 12, **dadurch gekennzeichnet, dass** man zwischen den Schritten c) und d) am dritten Tag, danach alle Tage, eine Zugabe von neuem Medium bewirkt.

14. Verfahren nach einem der Ansprüche 9 bis 13, **dadurch gekennzeichnet, dass** der Schritt d) durch enzymatische Behandlung, Waschen in einem Medium, das keinen Wachstumsfaktor enthält, und Zentrifugieren bewirkt wird.

15. Verfahren nach einem der Ansprüche 9 bis 14, **dadurch gekennzeichnet, dass** man am Ende von Schritt d) einen Schritt e) bewirkt, in dem die ES-Zellen auf einen Teppich von Nährzellen in Anwesenheit von Medium gemäß Anspruch 1 wieder eingeimpft werden, um eine sekundäre Kultur zu erhalten.

16. Verfahren nach Anspruch 15, **dadurch gekennzeichnet, dass** die Schritte d) und e) mehrere Male wiederholt werden.

17. Verfahren nach einem der Ansprüche 9 bis 16, **dadurch gekennzeichnet, dass** der Teppich aus Nährzellen aus mit Mitomycin behandelten oder bestrahlten STO-Zellen besteht.

18. In-vitro-Kultur von Vogel-ES-Zellen, erhältlich durch das Verfahren nach einem der Ansprüche 10 bis 17, wobei die Kultur ein Kulturmedium nach Anspruch 1 enthält.

19. Kultur von Vogel-ES-Zellen nach Anspruch 18, **dadurch gekennzeichnet, dass** sie die Kombination von SCF, b-FGF und LIF enthält, wobei die Kultur 2 bis 3 mal mehr für die alkalische Phosphatase-Aktivität positive Kolonien bezüglich der Nährbrühe aufweist, die zur Hauptsache aus schwach positiven Zellen besteht.

20. Kultur von Vogel-ES-Zellen nach Anspruch 18, **dadurch gekennzeichnet, dass** die Zellen durch Integration eines Gens modifiziert sind, das für ein heterologes Protein kodiert.

## Claims

1. Culture medium for avian totipotent embryonic cells, of the type containing an avian cell culture medium, **characterized in that** it comprises at least one compound chosen from cytokines, fibroblast growth factors, insulin-like growth factors and stem cell growth factors, and **in that** it comprises anti-retinoic acid antibodies (ARMA).

2. Culture medium according to Claim 1, **characterized in that** the cytokine is chosen from LIF (leukaemia inhibitory factor), IL-11 (interleukine-11), IL-6 (interleukine-6), CNTF (ciliary neurotrophic factor), oncostatin M (OSM) and mixtures thereof.

3. Culture medium according to either of Claims 1 and 2, **characterized in that** it contains at least one cytokine chosen from the group consisting of LIF, IL-11, IL-6, and the various mixtures thereof.

4. Culture medium according to one of Claims 1 to 3, **characterized in that** it contains a fibroblast growth factor which is b-FGF (basic fibroblast growth factor).

5. Culture medium according to one of Claims 1 to 4, **characterized in that** it contains an insulin-like growth factor which is IGF-1 (insulin-like growth factor-1).

6. Culture medium according to one of Claims 1 to 5, **characterized in that** it contains a stem cell growth factor chosen from a-SCF (avian stem cell factor) and m-SCF (murine stem cell factor).

7. Culture medium according to one of Claims 1 to 6, **characterized in that** it contains a lawn of feeder cells.

8. Culture medium according to one of Claims 1 to 7, **characterized in that** it also contains the following compounds:
- Foetal bovine serum
- Chicken serum
- Conalbumin
- Nonessential amino acids
- Sodium pyruvate
- Nucleoside stock
- Hepes (1 M)
- β-Mercaptoethanol
- Penicillin
- Streptomycin
- Gentamicin
with the nucleoside stock consisting of the mixture:
adenosine, guanosine, cytidine, uridine and thymidine in aqueous solution.

9. Method of culture of avian totipotent embryonic cells (or avian ES cells), **characterized in that**:
a) cells originating from blastoderm discs of fertilized eggs are suspended in an avian cell culture medium comprising at least one compound chosen from cytokines, fibroblast growth factors, insulin-like growth factors and stem cell growth factors, the said mixture being free from retinoic acid which is active for the action of the anti-retinoic acid antibodies present in the said medium,
b) a lawn of feeder cells is inoculated with the suspension obtained after step a),
c) the cells are incubated,
d) the cells in culture are removed and purified so as to recover bird ES cells.

10. Method according to Claim 9, **characterized in that**, between steps c) and d), one or more additions of fresh medium identical to the one used in step a) are performed at intervals of time.

11. Method according to either of Claims 9 or 10, **characterized in that** the medium of step a) contains the following components: b-FGF, a-SCF, IGF-1, LIF, IL-11, IL-6 and anti-retinoic acid antibodies.

12. Method according to one of Claims 9 to 11, **characterized in that** the medium of step a) contains, in addition, the following compounds:
- Foetal bovine serum
- Chicken serum
- Conalbumin
- Nonessential amino acids
- Sodium pyruvate
- Nucleoside stock
- Hepes (1 M)
- β-Mercaptoethanol
- Penicillin
- Streptomycin
- Gentamicin
with the nucleoside stock consisting of the mixture:
adenosine, guanosine, cytidine, uridine and thymidine in aqueous solution.

13. Method according to one of Claims 10 to 12, **characterized in that**, between steps c) and d), the addition of fresh medium is performed on day 3 and then every day.

14. Method according to one of Claims 9 to 13, **characterized in that** step d) is performed by enzymatic treatment, washing in a medium not containing any growth factor and centrifugation.

15. Method according to one of Claims 9 to 14, **characterized in that**, after step d), a step e) is performed in which the ES cells are reinoculated onto a lawn of feeder cells in the presence of the medium according to Claim 1 so as to obtain a secondary culture.

16. Method according to Claim 15, **characterized in that** the steps d) and e) are repeated several times.

17. Method according to one of Claims 9 to 16, **characterized in that** the lawn of feeder cells consists of mitomycin-treated or irradiated STO cells.

18. In vitro culture of avian ES cells which is capable of being obtained by the method according to one of Claims 10 to 18, the said culture comprising a culture medium according to Claim 1.

19. Culture of avian ES cells according to Claim 18, **characterized in that** it comprises the combination of SCF, b-FGF and LIF, the said culture having 2 to 3 times more colonies which are positive for the alkaline phosphatase activity relative to the baseline level consisting predominantly of weakly positive cells.

20. Culture of avian ES cells according to Claim 18, **characterized in that** the cells are modified by integration of the gene coding for a heterologous protein.
